(19) **Europäisches Patentamt / European Patent Office / Office européen des brevets**

(11) **EP 4 353 748 A1**

(12) **EUROPEAN PATENT APPLICATION**
published in accordance with Art. 153(4) EPC

(43) Date of publication:
**17.04.2024 Bulletin 2024/16**

(21) Application number: **22819527.7**

(22) Date of filing: **07.06.2022**

(51) International Patent Classification (IPC):
**C07K 16/36** (2006.01)   **C12N 15/13** (2006.01)
**A61K 39/395** (2006.01)   **A61P 7/02** (2006.01)

(52) Cooperative Patent Classification (CPC):
**A61K 39/395; A61K 47/64; A61K 47/65;**
**A61K 47/68; A61P 7/02; C07K 16/00; C07K 16/36;**
**C07K 19/00**

(86) International application number:
**PCT/CN2022/097412**

(87) International publication number:
**WO 2022/257929 (15.12.2022 Gazette 2022/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **08.06.2021   CN 202110639032**

(71) Applicant: SHANGHAI SYNVIDA
BIOTECHNOLOGY CO. LTD.
**Shanghai 201303 (CN)**

(72) Inventors:
• LIU, Junling
  **Shanghai 201203 (CN)**

• FAN, Xuemei
  **Shanghai 201203 (CN)**
• SUN, Tianyao
  **Shanghai 200060 (CN)**

(74) Representative: **Delbarba, Andrea**
**Bugnion S.p.A.**
**Viale Lancetti, 17**
**20158 Milano (IT)**

Remarks:
The complete document including Reference Table(s) and the Sequence Listing(s) can be downloaded from the EPO website

(54) **NOVEL ANTITHROMBOTIC ANTIBODY**

(57)    The present invention provides a novel antithrombotic antibody, the antibody uses FIXa as a target and has unique properties, and specifically targets the binding site of coagulation factor FIXa and FVIIIa, reduces the formation of a FVIIa-FIXa complex, blocks the conversion of FX to FXa, and thereby exerts an antithrombotic effect. The antibody of the present invention has suitable antithrombotic properties, and has a large effective therapeutic concentration window, but does not increase the risk of bleeding in addition, the antibody can meet the needs for suitable antithrombotic performance in clinical application and for effective avoidance of problems regarding bleeding caused by excessive effects. The present invention also discloses a method for screening drugs by using an FIXa-FVIIIa binding site as a target.

EP 4 353 748 A1

## Description

### TECHNICAL FIELD

The disclosure belongs to the fields of immunology and pharmacy. More specifically, the disclosure relates to a novel antithrombotic antibody, targeting the coagulation factor FIXa-FVIIIa binding site.

### BACKGROUND

Thromboembolic disease is a common clinical disease characterized by arteriovenous and microvascular thrombosis or embolism. Due to the obstruction of blood flow caused by thrombus formation or the interruption of downstream blood flow caused by emboli detachment, ischemia and necrosis of tissues and organs may occur. Globally, approximately 17.9 million people die from cardiovascular diseases, accounting for 31% of the global mortality rate. Thrombosis is a significant factor in triggering various severe cardiovascular and cerebrovascular diseases. Thromboembolic disease has been the primary cause of threats to human health and life in today's society. Main treatment approach for thromboembolic disease is antithrombotic therapy, including anticoagulation, antiplatelet and thrombolysis. Among them, anticoagulant therapy mainly targets different coagulation factors in coagulation cascade signaling pathway, blocking coagulation process by suppressing these factors.

Coagulation factors are various protein components involved in the process of blood clotting. Their physiological function is to be activated during vascular bleeding, adhere to platelets, and seal the leak in the blood vessel. This process is referred to as coagulation. The entire coagulation process can be roughly divided into two stages: the activation of prothrombin and the formation of gelatinous fibrin. For unified naming, the World Health Organization assigns Roman numerals to coagulation factors based on the order of their discovery, such as coagulation factors (F) I, II, III, IV, V, VI, VII, VIII, IX, X, XI, XII, XIII, and so on. Some coagulation factors have an "a" added to their number to denote their activated form. For example, the activated form of coagulation factor IX (FIX) is FIXa, and the activated form of coagulation factor VIII (FVIII) is FVIIIa.

Currently, anticoagulants used in clinical include heparin and its derivatives, vitamin K antagonists (such as warfarin), and small molecule inhibitors like rivaroxaban and dabigatran. All these drugs can act on the common coagulation pathway (e.g., factors Ha and Xa) in coagulation cascade. Due to their impacts on the physiological hemostatic function, there is a serious risk of bleeding, especially cerebral hemorrhage. Since the endogenous coagulation pathway is closely related to pathological thrombosis rather than hemostatic function, selective inhibitors of endogenous coagulation factors have become a hot spot in the research of novel anticoagulants.

FIXa is a critical coagulation factor in the endogenous coagulation pathway and is the only soluble form of coagulation protein. FIXa efficiently diffuses from tissue factor-bearing cells to platelets, serving as a crucial link between the initiation and amplification phases of the coagulation cascade. FIX can also be directly activated by FXIa on aggregated platelets. FIXa activates FX by forming a FIXa-FVIIIa complex. However, the binding sites of FVIIIa and FIXa and their binding effects are still unclear in this field.

### SUMMARY OF THE INVENTION

The object of the present disclosure is to provide a novel antithrombotic antibody targeting the binding site of coagulation factor FIXa-FVIIIa and the use thereof.

In the first aspect, the present disclosure provides an antithrombotic monoclonal antibody or antigen-binding fragment thereof, wherein the antithrombotic monoclonal antibody or antigen-binding fragment thereof has a heavy chain CDR1 with the amino acid sequence of SEQ ID NO: 3, a heavy chain CDR2 with the amino acid sequence of SEQ ID NO: 4, a heavy chain CDR3 with the amino acid sequence of SEQ ID NO: 5; and a light chain CDR1 with the amino acid sequence of SEQ ID NO: 6, a light chain CDR2 with the amino acid sequence of SEQ ID NO: 7, a light chain CDR3 with the amino acid sequence of SEQ ID NO: 8.

In a preferred example, the monoclonal antibody comprises: (a) a heavy chain variable region with the amino acid sequence of SEQ ID NO: 1 and a light chain variable region with the amino acid sequence of SEQ ID NO: 2; or (b) a heavy chain variable region with more than 80% (such as more than 85%, 90%, 93%, 95%, 97% or 99%) sequence identity to the amino acid sequence of SEQ ID NO: 1 and a light chain variable region with more than 80% (such as more than 85%, 90%, 93%, 95%, 97% or 99%) sequence identity to the amino acid sequence of SEQ ID NO: 2, also having function of the monoclonal antibody of (a).

In another preferred example, the monoclonal antibody comprises: murine antibody, chimeric antibody or humanized antibody; or, the monoclonal antibody or the antigen-binding fragment thereof comprises: single-chain antibody (scFV), domain antibody, Fab fragment , Fab' fragment, Fd fragment, F(ab')2 fragment.

In another preferred example, the antithrombotic monoclonal antibody or antigen-binding fragment thereof specifically targets the binding site of coagulation factor FIXa and FVIIIa, reduces the formation of a FVIIIa-FIXa complex, blocks the conversion of FX to FXa, and thereby exerts an antithrombotic effect.

In another preferred example, the antithrombotic monoclonal antibody or antigen-binding fragment thereof significantly prolonged the activated partial thromboplastin time (APTT) 2 to 4 times (such as 2.5, 3, 3.5 times; preferably using a normal time of activated partial thromboplastin time in natural body as the control, the normal time is for example 20-40 seconds, more preferably 25-36 seconds); or, the monoclonal antibody or antigen-binding fragment thereof prolonged the activated partial thromboplastin time (APTT) to more than 75 seconds, preferably more than 80 seconds (such as 80-120 seconds, more specifically such as 82, 85, 88, 90, 95, 100, 110 seconds).

In another preferred example, the antithrombotic monoclonal antibody or antigen-binding fragment thereof targets FIXa at the binding sites or adjacent sites of FIXa and FVIIIa, reduces the formation of a FVIIIa-FIXa complex; preferably, in FIXa, the binding sites or adjacent sites of FIXa and FVIIIa comprise: Asn93, Lys132, Arg165, Thr175; more preferably it also comprise: Ala95, Lys98, Asp164, Lys173, Tyr177; more preferably it also comprise: Lys126, Asn129, Asn178, Lys230, Arg233, Asn236.

In another preferred example, the amino acid residues at each site are numbered according to chymotrypsin.

In another preferred example, the antithrombotic monoclonal antibody or antigen-binding fragment thereof does not affect the catalytic activity of FIXa.

In another preferred example, the antithrombotic monoclonal antibody or antigen-binding fragment thereof does not bind to the catalytic site of FIXa, for example, the His57-Asp102-Ser195 site.

In another preferred example, the antithrombotic monoclonal antibody or antigen-binding fragment thereof does not affect prothrombin time (PT).

In another aspect, the present disclosure provides an isolated polynucleotide or a construct comprising the polynucleotide, the polynucleotide encodes any of the aforementioned antithrombotic monoclonal antibody or antigen-binding fragment thereof; preferably , the construct is an expression vector.

In another aspect, the present disclosure provides an antibody expression system, the expression system comprises the construct, or comprises the exogenous polynucleotide integrated in the genome; preferably, the expression system is a cell expression system.

In another aspect, the present disclosure provides a method for preparing the antithrombotic monoclonal antibody or antigen-binding fragment thereof, comprising: expressing the antibody by the antibody expression system under conditions suitable for expression; preferably it also comprises purifying and isolating the antibody.

In another aspect, the present disclosure provides a fusion protein, comprising the antithrombotic monoclonal antibody or the antigen-binding fragment thereof, and a fusion partner operably linked thereto; preferably, the fusion partner comprising (but are not limited to): a protein or an active structural domain having an effect of extending half-life in vivo, or a protein or an active structural domain having a synergistic function or binding effect on effectors (carry out another or more functions); more preferably, the protein or active structural domain having an effect of extending half-life in vivo comprises (but are not limited to): an immunoglobulin Fc region, preferably a human immunoglobulin Fc region, serum albumin (for example humanized HSA) or a fragment thereof.

In a preferred example, the immunoglobulin is selected from one or a combination of IgG, IgA1, IgA2, IgD, IgE, and IgM, wherein the IgG is selected from one or a combination of IgG1, IgG2, IgG3, or IgG4.

In another preferred example, there is a linker peptide between the antithrombotic monoclonal antibody or the antigen-binding fragment thereof and the fusion partner operably linked thereto; wherein the linker peptide is preferably selected from a flexible polypeptide chain composed of the group consisting of alanine and/or serine and/or glycine, wherein the length of the linker peptide is preferably 3-30 amino acids.

In another aspect, the present disclosure provides an immunoconjugate, wherein it comprises any of the aforementioned antithrombotic monoclonal antibody or the antigen-binding fragment thereof, or the aforementioned fusion protein; and the functional molecules linked (including but are not limited to covalently linked, coupled, attached, adsorbed) thereto; preferably, the functional molecules comprise (but are not limited to): molecules targeting surface markers on blood cells (such as platelet), hydrophilic polymers (such as PEG, PEGylated liposomes, etc.) or detectable markers (for example, including but not limited to: fluorescent markers, chromogenic markers).

In another aspect, the present disclosure provides a pharmaceutical composition, wherein the pharmaceutical composition comprises the antithrombotic monoclonal antibody or the antigen-binding fragment thereof, the fusion protein, or the immunoconjugate; preferably, the pharmaceutical composition further comprises a pharmaceutically acceptable carrier.

In another aspect, the present disclosure provides a use of the antithrombotic monoclonal antibody or antigen-binding fragment thereof, the fusion protein, or the immunoconjugate, or a pharmaceutical composition comprising them, in the manufacture of preparations, kits or drug kits for alleviating or treating thromboembolic diseases;

In a preferred example, the thromboembolic diseases comprise (but are not limited to): venous, arterial or capillary thrombosis, thrombosis in the heart, thrombosis during and/or after contacting blood with an artificial surface, interstitial lung disease (e.g. fibroproliferative and/or idiopathic pulmonary fibrosis), inflammation , neuro-inflammatory diseases, complement activation, fibrinolysis, angiogenesis, coagulation induced by FVIIIa-FIXa complex formation, coagulation induced by FX activation, coagulation induced by FIIa expansion, retinal vascular permeability-related diseases (e.g. embolism); preferably, diseases related to arterial or capillary thrombosis comprise (but are not limited to): myocardial infarction, stroke, deep vein thrombosis, portal vein thrombosis, renal vein thrombosis, jugular vein thrombosis, cerebral venous sinus thrombosis, Budd-Chiari syndrome or Paget-Schroetter disease.

In another aspect, the present disclosure provides a kit or drug kit, comprising the antithrombotic monoclonal antibody or the antigen-binding fragment thereof, the fusion protein, or the immunoconjugate or pharmaceutical compositions comprising them.

In another aspect, the present disclosure provides a method for screening antithrombotic substance (potential substance), comprising:

(1) Adding candidate substance to the system comprising FIXa and FVIIIa, wherein the FIXa and FVIIIa interact with each other (for example, they form a FVIIIa-FIXa complex);
(2) Detecting the interaction between FIXa and FVIIIa; if the candidate substance competes with FVIIIa to bind FIXa and reduces the formation of the FVIIIa-FIXa complex, it indicates that the candidate substance is a antithrombotic substance (including potential substance); preferably, the binding sites of the candidate substance and the FIXa complex can be predicted by protein-docking method for measuring the binding of FIXa and FVIIIa protein, more preferably, functions (competitive binding ability) of the candidate substance can be determined by observing the effect of the candidate substance in binding between FIXa and FVIIIa at the binding sites or adjacent sites of FIXa; more preferably, the binding sites or adjacent sites comprise: Asn93, Lys132, Arg165, Thr175; more preferably it also comprise: Ala95, Lys98, Asp164, Lys173, Tyr177; more preferably it also comprise: Lys126, Asn129, Asn178, Lys230, Arg233, Asn236; preferably, the amino acid residues at the sites can form a cluster, occupying the common surface between c170-helix and c131-helix of the FIXa protein.

In a preferred example, when observing the effect of the candidate substance in binding between FIXa and FVIIIa at the binding sites or adjacent sites of FIXa to determine the functions of the candidate substance, if the candidate substance exerts a strong binding effect (with significance), then it is a antithrombotic substance (including potential substance).

In another preferred example, the "reduction" (also referred to as inhibition, attenuation, etc.) is a statistically significant or significant reduction, such as a reduction of the FVIIIa-FIXa complex by 5%, 10%, 15%, 20%, 30%, 50%, 60%, 80%, 90%, 95% or more.

In another preferred example, a control group is also included, so as to clearly distinguish the difference between the interaction of FIXa and FVIIIa in testing group and that in the control group.

In another preferred example, the candidate substances comprise (but are not limited to): regulators designed for FIXa, or upstream or downstream proteins or genes thereof, such as antibodies, interfering molecules (such as interfering RNA), small molecular compounds, gene-modifying constructs or gene-editing constructs, and so on.

Other aspects of the present disclosure will be apparent to those skilled in the art based on the disclosure herein.

**DESCRIPTION OF FIGURES**

Figure 1A. Affinity of FIXa-4 antibody to FIXa.
Figure 1B. Activated partial thromboplastin time of FIXa-4 antibody.
Figure 1C. Prothrombin time of FIXa-4 antibody.
Figure 2. Effect of FIXa-4 antibody on FIXa catalytic activity.
Figure 3. Contact surface between FIXa-4 antibody and FIXa.

(A) Catalytic triad His57-Asp102-Ser195 (green; arrows) of FIXa (blue; left panel) was not blocked by the binding of FIXa-4 antibody (red; right panel).
(B) Contact surface between FIXa-4 antibody (red; right panel) and FIXa (blue; left panel) covered part of predicted binding sites between FIXa and FVIIIa.
(C) Back view of B, wherein the left panel is FIXa-4 antibody, right panel is FIXa, arrows showed part of predicted binding sites between FIXa and FVIIIa.

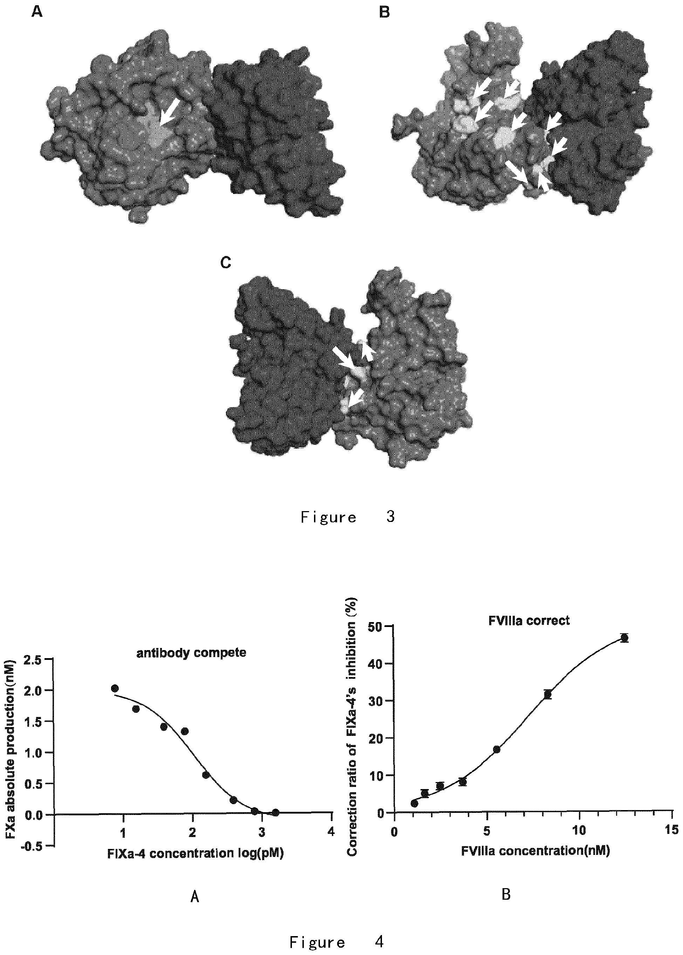

Figure 4A. Inhibition of FIXa-4 antibody on the production of FXa.
Figure 4B. Correction of FVIIIa in the inhibition of FIXa-4 antibody.

**DETAILED DESCRIPTION**

After in-depth research, the present inventors have revealed a high-affinity anti-FIXa antibody (FIXa-4) with unique properties targeting FIXa. Although the antibody does not bind to catalytic sites of the substrate of FIXa, it specifically targets the binding site of coagulation factor FIXa and FVIIIa, reduces the formation of a FVIIIa-FIXa complex, blocks the conversion of FX to FXa, and thereby exerts an antithrombotic effect. The antibody of the present disclosure has suitable antithrombotic properties, and has a large effective therapeutic concentration window, but does not increase the risk of bleeding. In addition, the antibody of the present disclosure does not directly bind to substrate catalytic sites of FIXa. Once excessive anticoagulation occurs, exogenous FVIII can be supplied for rescue, so as to meet the needs for suitable antithrombotic performance in clinical application and for effective avoidance of problems regarding bleeding caused by excessive effects.

**Terms**

As used herein, coagulation factor IX (sequence: GenBnak accession number: 2158) is also known as coagulation factor IX, coagulation factor 9, factor IX, FIX, F9, etc.; FIXa is the activated form of FIX. For example, with the participation of $Ca^{2+}$, FXIa cleaves FIX and turns it into activated FIX (FIXa). In some embodiments, variants are also comprised, for example, the variant protein has 1 or more (1-20; more specifically such as 2, 3, 4, 5 or 10) amino acid substitutions, deletions or insertions, and also remains the activity of FIX or FIXa.

As used herein, "antibody" or "immunoglobulin" are used as generic terms herein, comprising full length antibody, single chain antibody and all parts, domains or fragments thereof (including but are not limited to antigen binding domains or fragments). In addition, the term "sequence" as used herein (e.g. in terms of "immunoglobulin sequence", "antibody sequence", "single variable domain sequence", "VHH sequence" or "protein sequence", etc.) is to be understood generally comprising relevant amino acid sequence and the nucleic acid sequence or nucleotide sequence encoding the amino acid sequence, unless a more restrictive interpretation is required herein.

As used herein, "monoclonal antibody" refers to an antibody molecule preparation consisting of a single molecule. Monoclonal antibody exhibits a single-binding specificity and affinity for a specific epitope.

As used herein, "fusion partner", namely FP, Fusion Partner, refers to another polypeptide that is fused with the target polypeptide. The fusion partner can affect the functional properties of the fusion protein through a variety of different mechanisms, for example, prolonging in vivo half-life of the target polypeptide. For example, the fusion partners comprise, but are not limited to, a protein or an active structural domain having an effect of extending half-life in vivo, or a protein or an active structural domain having a synergistic function or binding effect on effectors, exerting another or more functions.

As used herein, "conjugate" refers to a product formed by functional molecules (including polypeptides, small molecular compounds, hydrophilic polymers, labels) covalent or non-covalent conjugated to the monoclonal antibody described herein, wherein the hydrophilic polymer and the polypeptide can be conjugated at any position, such as the N-terminal, C-terminal or a suitable position in the middle of the polypeptide. The hydrophilic polymer is, for example, polysaccharide, polyalkylene glycol, such as polyethylene glycol (PEG), polypropylene glycol (PPG), polyethylene oxide (PEO), copolymers of ethylene glycol and propylene glycol, polyvinyl alcohol, and so on.

As used herein, "antithrombotic" can also be interpreted as "increasing vascular permeability" or "anticoagulation".

"Sequence identity" between two polypeptide sequences indicates the percentage of identical amino acids between the sequences. "Sequence similarity" indicates the percentage of amino acids that are identical or have conservative amino acid substitutions. Methods for evaluating sequence identity between amino acids or nucleotides are known to those skilled in the art. For example, amino acid sequence identity is typically measured using sequence analysis software. For example, the program named BLAST of NCBI database can be used to determine identity.

An "effective amount" of an agent refers to the amount necessary to cause a physiological change in a cell or tissue after administration.

A "therapeutically effective amount" of an agent, such as a pharmaceutical composition, refers to an amount that can effectively achieve desired therapeutic or prophylactic results within the required dosage and timeframe. A therapeutically effective amount of an agent, for example, eliminates, reduces, delays, minimizes or prevents the adverse effects of a disease.

An "individual" or "subject" is a mammal. Mammals comprise, but are not limited to, domesticated animals (e.g., cows, sheep, cats, dogs and horses), primates (e.g., humans and non-human primates such as monkeys), rabbits, and rodents (e.g., mice and rats). Preferably, the individual or subject is a human.

The term "pharmaceutical composition" refers to a formulation in a form that allows the biological activity of the active ingredients contained therein is effective, without the inclusion of other components in the formulation that would impart unacceptable toxicity to the subjects receiving the composition.

"Pharmaceutically acceptable carrier" refers to ingredients other than the active ingredient in the pharmaceutical composition that are non-toxic to the subject. Pharmaceutically acceptable carriers comprise, but are not limited to, buffers, excipients, stabilizers or preservatives.

The term "treating/preventing" refers to altering natural course of a disease in a treated individual and may be a clinical intervention implemented for prophylaxis or during the course of clinical pathology. Desired effects of treatment comprise, but are not limited to, preventing the occurrence or recurrence of diseases, alleviating symptoms, reducing any direct or indirect pathological consequences of the disease, preventing metastasis, slowing the rate of disease progression, improving or mitigating the state of diseases, and preventing or improving prognosis.

The term "humanized antibody" refers to a molecule with antigen-binding sites substantially derived from an immunoglobulin of non-human species, wherein remaining structure of the immunoglobulin in the molecule is based on the structure and/or sequence of human immunoglobulin. The antigen-binding sites may comprise an entire variable domain fused to a constant domain, or it may only comprise complementarity determining regions (CDR) grafted into appropriate framework regions in the variable domain. The antigen-binding site may be wild-type, or modified by one or more amino acid substitutions, e.g., to more closely resemble with human immunoglobulin. Some forms of humanized antibody retain the entire complementarity determining region (CDR) sequences. Other forms have one or more CDRs that are altered relative to the original antibody..

The term "detectable marker" refers to a marker that can be attached to an antibody and is used to determine the presence or absence and the amount of a specific target in the subject. The "detectable marker" can be, but are not limited to: enzyme, fluorescent label, nuclide, quantum dot, colloidal gold, etc. More specifically, for example, it may be selected from: horseradish peroxidase (HRP), alkaline phosphatase (AP), glucose oxidase, $\beta$-D-galactosidase, urease, catalase, or glucoamylase.

**Antibody**

In the present disclosure, a monoclonal antibody was screened with the coagulation factor FIXa as the target, with its antithrombotic effect and mechanisms be studied. After extensive research and screening, an anti-FIXa antibody is provided. It specifically targets the binding site of coagulation factor FIXa and FVIIIa, reduces the formation of FVIIIa-FIXa complex, blocks the conversion of FX to FXa, and exerts an antithrombotic effect. The disclosure also comprises antigen-binding fragments of the anti-FIXa antibody.

By hybridoma technology, cell expression and purification for monoclonal antibody, the present inventor prepared a monoclonal antibody of high purity. The activated partial thromboplastin time (APTT) and prothrombin time (PT) were used to evaluate the antithrombotic effects of the monoclonal antibody. And then chromogenic substrate method was used to detect the effect of monoclonal antibody on the enzyme activity of FIXa. The method of protein-protein docking was adopted to predict the possible binding sites between FIXa and the antibody, and the binding site was verified by competitive assays (indirectly through the chromatic substrate method). The results showed that an anti-FIXa monoclonal antibody named FIXa-4 with high affinity was obtained, and FIXa-4 significantly prolonged APTT in a concentration-gradient dependence. Mechanistic studies found that FIXa-4 did not directly bind to substrate catalytic sites of FIXa, but occupied the binding region of FIXa and FVIIIa. Therefore, the present disclosure provides a novel monoclonal antibody, FIXa-4, the antibody competes with FVIIIa to bind FIXa, reduces the formation of a FVIIIa-FIXa complex, blocks the conversion of FX to FXa, and thereby exerts an antithrombotic effect.

In a preferred embodiment of the present disclosure, the anti-FIXa monoclonal antibody or antigen-binding fragment thereof has a heavy chain CDR1 with the amino acid sequence of SEQ ID NO: 3, a heavy chain CDR2 with the amino acid sequence of SEQ ID NO: 4, a heavy chain CDR3 with the amino acid sequence of SEQ ID NO: 5; and a light chain CDR1 with the amino acid sequence of SEQ ID NO: 6, a light chain CDR2 with the amino acid sequence of SEQ ID NO: 7, a light chain CDR3 with the amino acid sequence of SEQ ID NO: 8.

The anti-FIXa antibody provided by the present disclosure may comprise framework region FR, such as those listed in Table 1 below. However, the framework region is not limited to the sequences listed in Table 1. Modified antibodies, including partially or entirely altered sequences in the framework regions, are also encompassed within the scope of this disclosure. For example, chimeric antibody or humanized antibody formed by modifications are included.

The antigen-binding properties of an antibody are usually determined by the complementarity determining region CDR. The CDR region is arranged in an order with the FR region, and the FR region does not directly participate in binding. These CDR form a loop structure, and the $\beta$-fold created by adjacent FR that are close to each other in the spatial structure, constituting the antigen-binding site of the antibody. The CDR regions are sequences of proteins of interest in immunology, and the CDR regions of the antibody in the present disclosure are entirely novel.

The antibody of the present disclosure can be a complete immunoglobulin molecule or an antigen-binding fragment, including but are not limited to Fab fragment, Fd fragment, Fv fragment, F(ab')2 fragment, complementarity determining region (CDR) fragment, single chain antibody (scFv), domain antibody, bivalent single-chain antibody, single-chain phage antibody, bispecific diabody, triple-chain antibody, and quadruple-chain antibody.

In a preferred embodiment of the present disclosure, the anti-FIXa antibody has a heavy chain variable region with the amino acid sequence of SEQ ID NO: 1 and a light chain variable region with the amino acid sequence of SEQ ID NO: 2. The present disclosure also comprises: an antibody having a heavy chain variable region with more than 80% sequence identity to the amino acid sequence of SEQ ID NO: 1 and a light chain variable region with more than 80% sequence identity to the amino acid sequence of SEQ ID NO: 2, and also with the function of the antibody in the example of the present disclosure; preferably, in the heavy/light chain variable region, amino acids in the CDR region are conservative.

Functional variants of the antibody are included in the present disclosure. The variants can compete with the parental antibody for specific binding to FIXa, with an ability to recognize FIXa and a site for recognizing FIXa, wherein the site is close to those of the specific antibody provided in the examples of the present disclosure (targeting the binding site of blood coagulation factor FIXa-FVIIIa). The functional variants may have conservative sequence modifications, including nucleotide and amino acid substitutions, additions and deletions. These modifications may be introduced by standard techniques known in the art, such as site-directed mutagenesis and random PCR-mediated mutagenesis, and may comprise natural as well as unnatural nucleotides and amino acids. Preferably, the sequence modification occurs in regions other than the CDR regions of the antibody.

According to examples of the present disclosure, the antibody or antigen-binding fragment thereof of the present disclosure prolongs the activated partial thromboplastin time (APTT) about 3.5 times, with suitable antithrombotic properties. It has a large effective therapeutic concentration window, but not increase risk of bleeding.

**Constructs and Antibody Expression Systems**

The disclosure also provides a construct comprising the isolated polynucleotide of the disclosure. Methods for constructing the construct should be known to those skilled in the art. For example, the construct can be obtained by methods such as in vitro recombinant DNA technology, DNA synthesis technology, and in vivo recombination technology. More specifically, it can be constructed by inserting the isolated polynucleotide into the multiple cloning site of the expression vector. Expression vectors in the present disclosure generally refers to various commercially available expression vectors well known in the art,, such as bacterial plasmids, phages, yeast plasmids, plant cell viruses, mammalian cell viruses such as adenovirus, retrovirus or other vectors. The vector may also include one or more regulatory sequences operably linked to the polynucleotide sequence, and the regulatory sequences may include a suitable promoter sequence. The promoter sequence is usually operably linked to the coding sequence of the amino acid to be expressed. The promoter can be any nucleotide sequence that shows transcriptional activity in the host cell of choice, including mutated, truncated, and hybrid promoters, and can be derived from genes encoding extracellular or intracellular polypeptides homologous or heterologous to the host cell. The regulatory sequence may also be a suitable transcription termination sequence, a sequence recognized by a host cell to terminate transcription. A terminator sequence is linked to the 3' terminal of the nucleotide sequence encoding the polypeptide, and any terminator that is functional in the host cell of choice may be used in the present disclosure.

In general, suitable vectors may contain an origin of replication functional in at least one organism, a promoter sequence, convenient restriction sites and one or more selectable markers. For example, these promoters can include but are not limited to: the lac or trp promoter of *E. coli*; λ-phage PL promoter; eukaryotic promoters including the CMV immediate early promoter, the HSV thymidine kinase promoter, the early and late SV40 promoters, the LTR of retroviruses, and several other promoters known to control gene expression in prokaryotic or eukaryotic cells or their viruses. Marker genes may be used to provide phenotypic traits for selection of transformed host cells, for example, it may include but are not limited to dihydrofolate reductase for eukaryotic cell culture, neomycin resistance, and green fluorescent protein (GFP), or tetracycline or ampicillin resistance for *E. coli* ,etc. When the polynucleotide is expressed, an enhancer sequence can also be included in the expression vector. If the enhancer sequence is inserted into the vector, the transcription will be enhanced. The enhancer is a cis-acting factor of DNA, usually about 10 to 300 base pairs, with an act on the promoter to enhance gene transcription.

The present disclosure also provides an antibody expression system, wherein the expression system contains the construct of the present disclosure or the exogenous polynucleotide of the present disclosure integrated in the genome. Any cell suitable for expressing an expression vector can be used as a host cell. For example, the host cell can be a prokaryotic cell, such as a bacterial cell; or a lower eukaryotic cell, such as a yeast cell; or a higher eukaryotic cell, such as a mammalian cell. Specific examples include but are not limited to one or a combination of: bacterial cells of *Escherichia coli, Streptomyces, Salmonella typhimurium*; fungal cells such as yeast cells, filamentous fungi cells, plant cells; insect cells of Drosophila S2 or Sf9; CHO, COS, HEK293 cells, or animal cells of Bowes melanoma cells, etc. Methods for constructing the expression system should be known to those skilled in the art, for example, it may include but not limited to one or a combination of: microinjection, particle bombardment, electroporation, virus-mediated transformation, electroporation, calcium phosphate precipitation, etc.

**Fusion protein/immunoconjugate**

The present disclosure also includes a fusion protein comprising the first domain of the antibody according to the disclosure and the second domain for prolonging the half-life in vivo and/or binding to effector molecules or cells.

In the second domain, fragments for prolonging half-life in vivo may include serum albumin or its fragments, domains that bind serum albumin (such as anti-serum albumin antibodies), and so on.

In the second domain, the fragments capable of binding to effector molecules or cells may include immunoglobulin Fc regions, etc., preferably selected from human immunoglobulin Fc regions. The human immunoglobulin Fc region includes mutations for changing Fc-mediated effector functions, and the effector functions include one or more activities of CDC, ADCC and ADCP. The immunoglobulin may be selected from one or a combination of IgG, IgA1, IgA2, IgD, IgE, and IgM, wherein the IgG may be specifically selected from one or a combination of IgG1, IgG2, IgG3, or IgG4. The immunoglobulin Fc region contained in the antibody fusion protein can enable the fusion protein to form a dimer, also prolong in vivo half-life of the fusion protein and increase Fc-mediated related activities. In a specific embodiment of the present disclosure, the immunoglobulin Fc region can be the Fc region of human IgG1, more specifically, it can be the wild-type IgG1 Fc sequence, and the sequence can be introduced with a mutation for changing the effector function mediated by Fc, for example, a) a mutation that alters Fc-mediated CDC activity; b). a mutation that alters Fc-mediated ADCC activity; or c). a mutation that alters Fc-mediated ADCP activity. Such mutations are described in: Leonard G Presta, Current Opinion in Immunology 2008, 20:460-470; Esohe E.Idusogie et al., J Immunol 2000, 164:4178-4184; RAPHAEL A. CLYNES et al., Nature Medicine, 2000, Volume 6, Number 4:443-446; Paul R. Hinton et al., J Immunol, 2006, 176:346-356.

In the fusion protein of the anti-FIXa antibody provided by the present disclosure, a linker peptide may be provided between the first domain and the second domain. The linker peptide is a flexible polypeptide chain composed of alanine (A) and/or serine (S) and/or glycine (G), wherein the length of the linker peptide may be 3-30 amino acids, preferably 3-9 amino acids, 9-12 amino acids, 12-16 amino acids, 16-20 amino acids. In another specific embodiment of the present disclosure, the length of the linker peptide may be 8 or 15 amino acids.

The present disclosure also provides an isolated polynucleotide encoding the antibody of the present disclosure, or encoding the fusion protein, and the polynucleotide, wherein the polynucleotide may be RNA, DNA or cDNA, etc. Methods for providing the isolated polynucleotide should be known to those skilled in the art, for example, it can be prepared by automatic DNA synthesis and/or recombinant DNA technology, or it can be isolated from a suitable natural source.

The present disclosure also provides an immunoconjugate comprising the antibody of the present disclosure or the fusion protein of the present disclosure. Generally, the immunoconjugate also comprises a functional molecule linked (including but not limited to covalently linked, coupled, attached, adsorbed) to the antibody or fusion protein, wherein the functional molecule may be included but not limited to one or a combination of hydrophilic polymers, detectable markers, radioisotopes, bioactive proteins, molecules targeting surface markers on blood cells (such as platelet).

The hydrophilic polymers include but are not limited to: polyethylene glycol, PEGylated liposomes, polysaccharide, polyalkylene glycol, polypropylene glycol (PPG), polyethylene oxide (PEO), copolymers of ethylene glycol and propylene glycol, polyvinyl alcohol, etc., or any combination thereof.

Methods for preparing the immunoconjugate should be known to those skilled in the art, for example, the antibody and/or fusion protein can be linked directly or through a spacer with suitable length to the functional molecule by chemical cross-linking or genetic engineering fusion expression, so as to obtain the immunoconjugate.

The immunoconjugate may comprise an antibody or fusion protein of the disclosure and a detectable marker. The detectable markers include but are not limited to: fluorescent markers, chromogenic markers, protein labels; such as: enzymes, prosthetic groups, fluorescent materials, luminescent materials, bioluminescent materials, radioactive materials, positron-emitting metals and non-radioactive paramagnetic metal ions. More than one marker may also be included. Markers used to label the antibody for detective and/or analytical and/or diagnostic purposes depends on the particular detective/analytical/diagnostic techniques and/or methods, for example, immunohistochemical staining of (tissue) samples, flow cytometry, etc. Suitable markers are well known to those skilled in the art for detective/analytical/diagnostic techniques and/or methods known in the art.

Antibodies or fusion proteins of the disclosure can be coupled to a labeling group (labeled polypeptide), which can then be used, for example, for diagnostic purposes. Suitable labeling groups may be selected from radioisotopes (such as those mentioned above) or groups containing radioisotopes, radionuclides, fluorescent groups (such as fluorescent proteins such as GFP, RFP, etc., dyes, rhodamine, fluorescent cyanine and its derivatives such as FITC, cyanine dyes), enzyme groups (such as horseradish peroxidase, alkaline phosphatase, $\beta$-galactosidase), chemiluminescent groups, biotin groups, metal particles (e.g. gold particles), magnetic particles (e.g. having a core containing magnetite ($Fe_3O_4$)) and/or maghemite ($Fe_2O_3$), predetermined polypeptide groups, etc.

The immunoconjugate may comprise an antibody or fusion protein of the disclosure and a molecule targeting surface markers on blood cells such as platelets. The molecules targeting surface markers on blood cells can recognize blood cells, facilitating the antibodies of the disclosure to reach blood cells.

**Pharmaceutical composition and kit**

The present disclosure also provides a pharmaceutical composition, comprising the anti-FIXa antibody of the present disclosure, or the fusion protein of the anti-FIXa antibody of the present disclosure, or the immunoconjugate of the present disclosure.

Various pharmaceutically acceptable carriers in the art may also be included in the pharmaceutical composition. The pharmaceutically acceptable carrier is non-toxic to the subject at the dose and concentration used, and may specifically include but not limited to: buffers such as acetate, tris, phosphate, citrate and other organic acids; antioxidants, including ascorbic acid and methionine; preservatives (such as stearyldimethylbenzyl ammonium chloride; hexadimonium chloride; benzalkonium chloride, benzethonium chloride; phenol, butanol, or benzyl alcohol; hydrocarbylparabens, such as methylparaben or propylparaben; catechol; resorcinol; cyclohexanol; 3-pentanol; and m-cresol); proteins, such as serum albumin, gelatin or immunoglobulin; hydrophilic polymers such as polyvinylpyrrolidone; amino acids such as glycine, glutamine, asparagine, histidine, arginine or lysine; monosaccharide, disaccharide and other carbohydrates, including glucose, mannose, or dextrin; chelating agents, such as EDTA; tonicity modifiers, such as trehalose and sodium chloride; sugars, such as sucrose, mannitol, trehalose or sorbitol; surfactants such as polysorbate, salt-forming counterions, such as sodium; metal complexes (such as Zn-protein complexes); and/or nonionic surfactants, such as TWEE®, PLURONICS®, or polyethylene glycol (PEG). Pharmaceutical preparations for in vivo administration are generally sterile. Methods for achieving sterility of pharmaceutical preparations should be known to those skilled in the art, for example, they can be achieved by methods such as filtration through sterile membranes. Those skilled in the art can also select a suitable pharmaceutically acceptable carrier based on the formulation requirements of the pharmaceutical composition to prepare it in various forms. For example, the pharmaceutical composition of the present disclosure can include but not limited to tablets, injections, lyophilized preparations and other forms.

In the pharmaceutical composition, the content of the monoclonal antibody, fusion protein or immunoconjugate is usually an effective amount, and the content of the active ingredient corresponding to the effective amount can be determined according to the object to be treated and the specific administration method. For example, based on the total mass of the pharmaceutical composition, the content of the monoclonal antibody, fusion protein and immunoconjugate may be about 0.01-99%, 0.1-70%, 1-30%, 0.01-0.05%, 0.05-0.1%, 0.1-0.3%, 0.3-0.5%, 0.5-1%, 1-3%, 3-5%, 5-10%, 10-20%, 20-30%, 30-50%, 50-70%, or 70-99%.

The monoclonal antibody, fusion protein, and immunoconjugate of the present disclosure can be administered as a single active ingredient, or in combination therapy, that is, in combination with other agents. For example, the combination therapy may be the monoclonal antibody, fusion protein, immunoconjugate combined with at least one other antithrombotic drug. For another example, the combination therapy may be the combined use of the monoclonal antibody, fusion protein, immunoconjugate and antibodies targeting specific antigens on other blood cells.

The present disclosure also provides a detection kit, comprising the antibody, fusion protein or immunoconjugate of the present disclosure. The kit may also include: containers, controls (negative or positive controls), buffers, auxiliary agents, etc., which can be selected by those skilled in the art according to specific conditions. Instructions may also be included in the kit, so as to facilitate the operation of those skilled in the art.

**The use**

The present disclosure also provides a use of the antibody, fusion protein, immunoconjugate or pharmaceutical composition of the present disclosure in the manufacture of preparations, kits or drug kits for alleviating or treating thromboembolic diseases.

In the present disclosure, the "thromboembolic diseases" comprise: venous, arterial or capillary thrombosis, thrombosis in the heart, thrombosis during and/or after contacting blood with an artificial surface, interstitial lung disease (e.g. fibroproliferative and/or idiopathic pulmonary fibrosis), inflammation, neuro-inflammatory diseases, complement activation, fibrinolysis, angiogenesis, coagulation induced by FVIIIa-FIXa complex formation, coagulation induced by FX activation, coagulation induced by FIIa expansion, retinal vascular permeability-related diseases (e.g. embolism), etc.

Wherein, diseases related to arterial or capillary thrombosis comprise (but are not limited to): myocardial infarction, stroke, deep vein thrombosis, portal vein thrombosis, renal vein thrombosis, jugular vein thrombosis, cerebral venous sinus thrombosis, Budd-Chiari syndrome or Paget-Schroetter disease.

A "therapeutically effective amount" of the fusion protein, immunoconjugate, and pharmaceutical composition provided in the present disclosure preferably leads to a reduction in the severity of disease symptoms, an increase in the frequency and duration of the asymptomatic phase of the disease, or prevention of damage or disability caused by the disease. For example, for the treatment of thrombotic disorders, a "therapeutically effective amount" preferably reduces thrombus formation (or increases vascular permeability) by at least about 10%, preferably at least about 20%, more preferably at least about 30%, more preferably at least about 40%, more preferably at least about 50%, more preferably at least about 60%, more preferably at least about 70%, more preferably at least about 80% relative to an untreated subject or a non-diseased period in the same subject. The ability to inhibit thrombus formation can be evaluated in in vitro reaction systems, cell models or animal model systems. Those skilled in the art can select an appropriate therapeutically effective amount according to the actual situation, for example, the size of the subject, the severity of the subject's symptoms and the selected specific composition or administration route. Prescriptions for treatment (e.g., decisions on dosage, etc.) can be determined by a doctor, considering factors including but not limited to the disease being treated, individual patient conditions, administration site, method of administration, and other factors. A prophylactically effective amount refers to an amount effective at the dosage and time necessary to achieve the desired prophylactic effect. Usually, but not necessarily, a "prophylactically effective amount" is generally lower than a "therapeutically effective amount" because the prophylactic dose is administered to the subject before the onset of the disease or at an early stage of the disease.

The present disclosure further provides a detection method for detecting FIXA antigen using the antibody, including but not limited to qualitative detection, quantitative detection and localization detection. Specifically, the detection methods include but are not limited to immunofluorescence, immunohistochemistry, radioimmunoassay and so on.

A method for detecting the presence of FIXa antigen in a sample may include: contacting the sample with the antibody of the present disclosure; observing whether an antibody complex is formed, and the formation of an antibody complex indicates the presence of FIXa antigen in the sample. The sample can be a cell and/or tissue sample; the sample can be fixed or dissolved in a medium; and the level of FIXa antigen in the fixed or dissolved sample can be detected. In some embodiments, the objects for detection may be a cell-containing sample present in a cell preservation solution. In other embodiments, the antibody is also conjugated with markers that can be used for detection or can be detected by other reagents, such as fluorescent dyes, chemicals, polypeptides, enzymes, isotopes, labels, etc.

In the present disclosure, the FIXa-4 antibody is different from the previously reported FIXa inhibitors in that it does not directly act on the catalytic active site of FIXa, but occupies most of the binding region between FIXa and FVIIIa, blocking the formation of the FIXa-FVIIIa complex, then affects the conversion of FX to FXa and triggers anticoagulation. The inventors also observed that the FIXa-4 antibody substantially blocked the production of FXa in vitro, and supplementation with FVIIIa corrected this inhibition. That is, the antithrombotic effect of FIX-4 antibody was partially reversed with the increase of FVIIIa concentration. Compared with the existing antithrombotic drugs, the reversible antithrombotic effect needs to be considered in the development of novel safe and effective antithrombotic drugs. This feature can be used in clinical medication to prevent or treat uncontrollable bleeding side effects caused by excessive anticoagulation. Therefore, this discovery by the present inventors is particularly meaningful. It can be referenced how the FIXa-4 antibody interacts with FIXa to screen or design a class of drugs that can achieve similar competitive binding. These drugs may potentially be drugs with both coagulation-regulating effect and safety.

Therefore, based on the new findings, the present inventors provide a method for screening antithrombotic substance, comprising: 1) adding candidate substance to the system comprising FIXa and FVIIIa, wherein the FIXa and FVIIIa interact with each other (for example, they form a FVIIIa-FIXa complex); (2) detecting the interaction between FIXa and FVIIIa; if the candidate substance competes with FVIIIa to bind FIXa and reduces the formation of the FVIIIa-FIXa complex, it indicates that the candidate substance is a antithrombotic substance; preferably, functions of the candidate substance can be determined by observing the effect of the candidate substance in binding between FIXa and FVIIIa at the binding sites or adjacent sites of FIXa; the binding sites or adjacent sites comprise: Asn93, Lys132, Arg165, Thr175; more preferably it also comprise: Ala95, Lys98, Asp164, Lys173, Tyr177; more preferably it also comprise: Lys126, Asn129, Asn178, Lys230, Arg233, Asn236. The amino acid residues at the sites can form a cluster, occupying the common surface between c170-helix and c131-helix of the FIXa protein.

Further, when observing the effect of the candidate substance in binding between FIXa and FVIIIa at the binding sites or adjacent sites of FIXa to determine the functions of the candidate substance, if the candidate substance exerts a strong binding effect, then it is a antithrombotic substance.

Methods for screening substances acting on a protein or gene or its specific region as a target are well known to those skilled in the art, and these methods can be used in the present disclosure. The candidate substances can be selected from: peptides, polymeric peptides, peptidomimetics, non-peptide compounds, carbohydrates, lipids, antibodies or antibody fragments, ligands, small organic molecules, small inorganic molecules, nucleic acid sequences, and so on. Based on the type of substances to be screened, it is clear to those skilled in the art how to select a suitable screening method. In some relatively specific examples, the candidate substances comprise but are not limited to: regulators designed for FIXa, or upstream or downstream proteins or genes thereof, such as antibodies, interfering molecules (such as interfering RNA), small molecular compounds, gene-modifying constructs or gene-editing constructs, and so on.

After large-scale screening, a class of potential substances that specifically act on the above-mentioned sites of interest with regulatory effects can be obtained.

The disclosure if further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

When numerical ranges are given in the embodiments, it should be understood that, unless otherwise indicated in the present disclosure, any value between the two endpoints of each numerical range and any value within the range can be selected. Unless otherwise defined, all technical and scientific terms used in the present disclosure have the same meaning as understood by those skilled in the art. In addition to the specific methods, devices, and materials used in the embodiments, those skilled in the art, based on their understanding of the prior art and the disclosure of the present disclosure, may also use any methods, devices, and materials similar or equivalent to those described in the embodiments to practice the present disclosure.

## 1 Materials and Methods

### 1.1 Preparation of a monoclonal antibody targeting FIXa

#### 1.1.1 Mice immunization and cell fusion

4 SPF-level BALB/c male mice (purchased from the Animal Science Department of Shanghai Jiao Tong University School of Medicine) at the age of 6-8 weeks were used for immunization. For the first immunization, purchased full-length 46KD FIXa antigen (Enzyme Research Laboratories) was mixed with complete Freund's adjuvant (Sigma) until the final concentration of FIXa was 100 $\mu$g/100 $\mu$l. 50 $\mu$g/50 $\mu$l of the mixture was injected into each of the two foot pads. For the second immunization, complete Freund's adjuvant was replaced by incomplete Freund's adjuvant (Sigma). For the third immunization, the FIXa antigen was diluted to 100 $\mu$g/100 $\mu$l with PBS and injected intraperitoneally. The fourth immunization, also known as booster immunization, was administered via tail vein injection. A total of 4 immunizations were carried out, with an interval of 2 weeks between each immunization.

On the 3rd day after the booster immunization, splenocytes of the immunized mice were fused with myeloma cells (laboratory cell line SP2/0) under the action of PEG to obtain hybridoma cells.

#### 1.1.2 Screening and cloning of antibody-positive cell lines

After culturing hybridoma cells for 7 days, ELISA was used for detecting positive antibodies. 0.1$\mu$g/100$\mu$l of FIXa antigen was coated on an ELISA plate (CORNING), incubated at 37°C for 1.5h; then blocked with 2% BSA at 37°C for 1h; and then primary antibody, the supernatant of cultured cells, was added and incubated at 37°C for 1.5h. After that, mouse secondary antibody was added and incubated at 37°C for 30 min. Finally, chromogenic substrate TMB (Thermo) was added for 5 min at room temperature in dark, then stop solution 2M $H_2SO_4$ was added to terminate the reaction. Antibody-positive cell lines were screened according to the absorbance value measured at 450 nm by a spectrophotometer (Thermo Fisher scientific company). Then the cell lines were cultured for cloning in a semi-solid medium (STEMCELL company), with single clones selected into 96-well plates after 7 days. Following the ELISA method described above, a subsequent screening was conducted, ultimately obtaining antibody-positive single clones.

#### 1.1.3 Antibody Sequencing

Sufficient amount of cell pellets was collected for RNA extraction, and then the RNA was reverse transcribed into cDNA. After that, appropriate primers were selected for PCR, and finally the PCR products were sent to the company for sequencing.

#### 1.1.4 Ascites preparation and antibody purification

4 male nude mice (purchased from the Animal Science Department of Shanghai Jiao Tong University School of Medicine) at the age of 6-8 weeks were selected. Pristane (Sigma company) was injected at a dose of 0.5ml/mouse 7 days in advance, followed by 0.5-1$\times$10$^6$/0.5ml of hybridoma cells intraperitoneally injected into the mice. After 7-10 days, abdominal distension was observed in the nude mice. A needle was inserted into the left lower abdominal cavity to collect ascites into EP tubes. After centrifuging at 5000 rpm for 10 minutes, the supernatant was collected, with NaN$_3$ added to a final concentration of 0.02%.

Following the protocol outlined in the manual for Protein G Agarose Resin (YEASEN), the ascites collected above was equilibrated, loaded, washed and eluted in sequence. Finally, the eluate was concentrated by ultrafiltration with a 30KD ultrafiltration tube to obtain high-purity monoclonal antibodies.

**1.2 Antibody subtype and affinity detection**

Following the instructions provided in the Sigma company's subtype detection kit, the antigen was coated, blocked, incubated with primary antibody (0.1$\mu$g/100$\mu$l), incubated with isotype-specific reagent at room temperature for 30 minutes, incubated with secondary antibody (R-antiGoat-HRP) for chromogen development, with stop solution added to terminate the reaction, and finally the absorbance was measured at 450nm. Affinity detection of the antibody is the same as the above ELISA method.

**1.3 Coagulation assay**

Antibody coagulation was assessed by activated partial thromboplastin time (APTT) and prothrombin time (PT).

**1.3.1 Activated partial thromboplastin time (APTT)**

Firstly, instruments and related reagents were preheated to 37°C. Then the antibody was diluted with OVB buffer (SIEMENS), then the APTT program was selected, and the cuvette was placed. 50 $\mu$l of APTT reagent, 50 $\mu$l of a mixture with plasma and magnetic beads were added in sequence. By pressing the start button, magnetic beads started to oscillate. Then 25 $\mu$l of antibody was added, with timing button pressed to start timing. After 180 seconds, 25 $\mu$l of 50 mM $CaCl_2$ (SIEMENS) was added, simultaneously the measurement button on the handle was pressed. Magnetic bead oscillation was observed until the plasma coagulates, with the seconds displayed on the instrument recorded. Finally, a curve was drawn according to the log value of the final antibody concentration in plasma and the coagulation time in seconds.

**1.3.2 Prothrombin time (PT)**

Firstly, instruments and related reagents were preheated to 37°C. Then the antibody was diluted with a mixture of plasma, then the PT program was selected, and the cuvette was placed. Magnetic beads were added for oscillation by pressing the start button, then 50$\mu$l of the mixture of plasma containing antibodies was added, with the timer button pressed to start timing. After 60s, 100$\mu$l PT reagent was added, simultaneously the measurement button on the handle was pressed. Magnetic bead oscillation was observed until the plasma coagulates, with the seconds displayed on the instrument recorded. A curve was drawn according to the log value of the final antibody concentration in plasma and the seconds.

**1.4 Effect of enzyme activities by FIXa chromogenic substrate method for detecting antibodies**

Firstly, the spectrophotometer was set to 37°C, and the kinetic cycle was selected to a total time of 10 min, an interval of 5s and 121 cycles. Then, following the instructions of FIXa chromogenic substrate SPECTROZYME (from IMMBIOMED), the following substances were added sequentially to a 96-well plate: 100$\mu$l Tris Buffer (50mM TRIS, 100mM NaCl, 5mM $CaCl_2$, pH7.4, 33% ethylene glycol), 10$\mu$l of 2$\mu$M FIXa protein, 2.5$\mu$l of antibody (with a final concentration of 1000, 10, 1, 0.1, 0.01$\mu$g/ml), and control TBS. Finally, 12.5$\mu$l of 10mM FIXa chromogenic substrate was added to trigger the reaction. $\Delta$OD/min of the absorbance value was measured at 405nm.

**1.5 Prediction of the binding site for the interaction between FIXa and FIXa-4 monoclonal antibody**

**1.5.1 Establishment of FIXa model**

The three-dimensional model of FIXa was constructed based on the crystal structure of an inhibitor complex of the catalytic domain of FIXa (PDBentry: 3LC3).

**1.5.2 Establishment of FIXa-4 monoclonal antibody model**

By Abody Builder of SabPred (Venkateswarlu D. Structural insights into the interaction of blood coagulation co-factor VIIIa with factor IXa: a computational protein-protein docking and molecular dynamics refinement study. Biochem Biophys Res Commun-2014:452(26) 14.), three-dimensional structure of the antibody variable region was predicted.

**1.5.3 Antibody-FIXa docking**

The antibody was docked with FIXa through ClusPro. During the docking, the antibody mode was used to shield the non-CDR region of the antibody (Dunbar J et al, SAbPred: a structure-based antibody prediction server. Nucleic Acids Res. 2016 Jul 8; 44(W1):W474-8).

### 1.5.4 Description of Antibody-FIXa Interaction

Diagrams describing the interaction of FIXa with antibodies were made using open-source PyMOL (version 2.5.0).

### 1.6 FIXa-4 antibody competes with FVIIIa for binding to FIXa

### 1.6.1 Activation of FVIII

100 μl 10 U/ml thrombin and 25 μl 4 mg/ml FVIII were added to 125 μl Buffer (20 mM Hepes, 300 mM NaCl, 2.5 mM CaCl 2 ), and incubated at 37° C for 10 min to obtain FVIIIa (with a final concentration of 0.4 mg/ml). Finally, 1U Hirudin was added to prevent the disintegration of FVIIIa.

### 1.6.2 Antibody inhibits the FX activation by FIXa-FVIIIa complex

Firstly, 50ng/2μl phosphatidylcholine (PC), phosphatidylserine (PS) mixture, 5μl 20nM FIXa and 10μl 3μM FX were mixed, then the premixed 8μl FVIIIa at a concentration of 80μg/ml and 5μl antibody were added and incubated at 37°C for 15min. Subsequently, 20 μl of 20 mM EDTA was added to terminate the reaction. Finally, 150 μl of TBS-Ca$^{2+}$ buffer containing 0.1% PEG8000 was added to dilute the reaction product.

Then, the spectrophotometer was set to 37°C, and the kinetic cycle was selected to a total time of 5min, an interval of 10s and 31 cycles. 40μl of the reaction product was added to a 96-well plate, and then 40μl of chromogenic substrate S2765 (1 mM) was added to trigger the reaction. ΔOD/min of the absorbance value was measured at 405nm.

### 1.6.3 Inhibition of FVIIIa-correcting antibody on FX activation by FIXa-FVIIIa complex

Appropriate antibody concentration in reaction 1.6.2 was selected. Following the same method, 5μl of antibody in the above reaction system was replaced with 2.5μl FVIIIa and 2.5μl of the antibody (with the final concentration remaining unchanged). ΔOD/min of the absorbance value was measured at 405nm.

### 1.6.4 Establish a standard curve between the amount of produced FXa and cleavage rate of the substrate

Firstly, 25μl FX (200nM), 25μl Russell Viper Venom (Russell Viper Venom, RVV) (42nM), 50μl TBS-Ca$^{2+}$ buffer (20mM Tris-HCl, 100mM NaCl, 5mM CaCl$_2$, BSA 0.1%) were mixed. After incubation at 37°C for 30 min, FX was activated to FXa (with a final concentration of 10 nM).

Then, the spectrophotometer was set to 37°C, and the kinetic cycle was selected to a total time of 5min, an interval of 10s and 31 cycles. 40μl of FXa in a concentration gradient was added to a 96-well plate, and then 40μl of chromogenic substrate S2765 (1 mM) was added to trigger the reaction. ΔOD/min of the absorbance value was measured at 405nm. That is the substrate cleavage rate. A standard curve was drawn according to the final concentration of FXa and the corresponding reaction rate.

### Example 1. The acquisition of an antibody

By mice immunization, hybridoma fusions, antibody sequencing, cell expression and purification, then after a large number of research and screening, the inventors obtained a monoclonal antibody with high affinity to human FIXa.

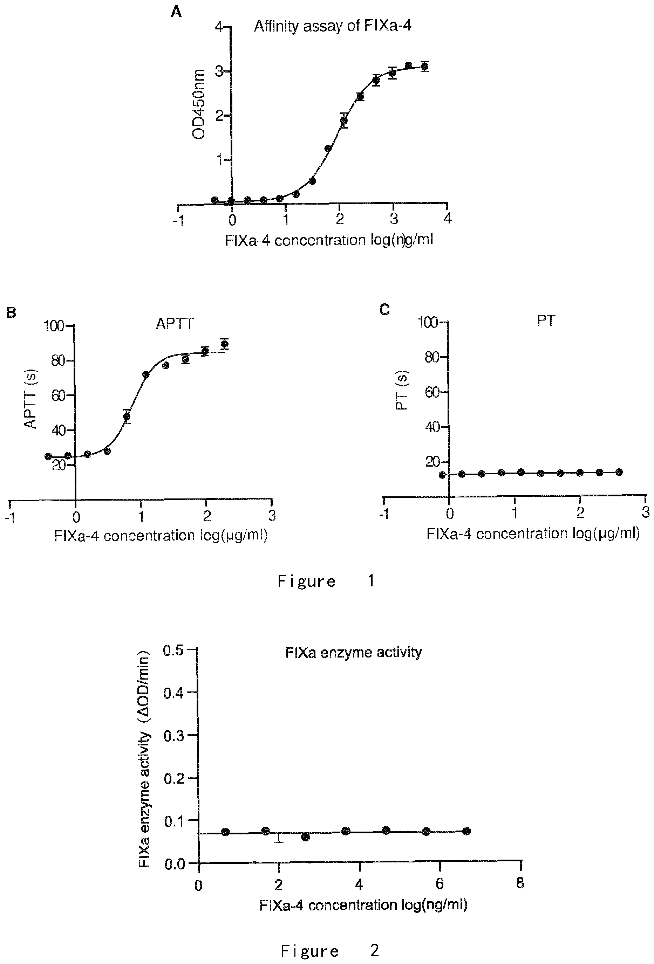

The half-maximum effect concentration (EC50) of the antibody was determined, and the result is shown in Figure 1A, EC50=94.67ng/ml.

The present inventors named this monoclonal antibody FIXa-4 antibody.

The amino acid sequence of the heavy chain variable region of the FIXa-4 antibody is as follows (SEQ ID NO: 1):

QVTLKESGPGILKPSQTLSLTCSFS**GFSLNTPGMG**VGWIRQPSGKGLEWLAH**IWW DDDK**YYNPSLKSQLTISKDTSRNQVFLKITSVDTADTATYYC**ARSDDVSYALDY**W GQGTSVTVSS

The amino acid sequence of the light chain variable region of the FIXa-4 antibody is as follows (SEQ ID NO: 2):

DIQMTQSPASLSASVGETVTITCRAS**ENIDSY**LAWYQQKQGKSPQLLVY**NAK**TLA
DGVPSRFSGSGSGTQFSLKIDSLQPEDFGSHYC**QHHDGTTWT**FGGGTKLEIK

Among them, the sequences of the CDR region and surrounding framework regions are listed in Table 1.

Table 1

| | FR1-IMGT | CDR1-IMGT | FR2-IMGT | CDR2-IMGT | FR3-IMGT |
|---|---|---|---|---|---|
| Heavy chain | QVTLKESGPGILKPSQTLSLTCSFS (SEQ ID NO: 9) | GFSLNTPGMG (SEQ ID NO: 3) | VGWIRQPSGKGLEWLAH (SEQ ID NO: 10) | IWWDDDK (SEQ ID NO: 4) | YYNPSLKSQLTISKDTSRNQVFLKITSVDTADTATYYC (SEQ ID NO: 11) |
| Light chain | DIQMTQSPASLSASVGETVTITCRAS (SEQ ID NO: 15) | ENIDSY (SEQ ID NO: 6) | LAWYQQKQGKSPQLLVY (SEQ ID NO: 16) | NAK (SEQ ID NO: 7) | TLADGVPSRFSGSGSGTQFSLKIDSLQPEDFGSHYC (SEQ ID NO: 17) |

| | CDR3-IMGT | JUNCTION | J-REGION | FR4-IMGT |
|---|---|---|---|---|
| Heavy chain | ARSDDVSYALDY (SEQ ID NO: 5) | CARSDDVSYALDYW (SEQ ID NO: 12) | YALDYWGQGTSVTVSS (SEQ ID NO: 13) | WGQGTSVTVSS (SEQ ID NO: 14) |
| Light chain | QHHDGTTWT (SEQ ID NO: 8) | CQHHDGTTWTF (SEQ ID NO: 18) | WTFGGGTKLEIK (SEQ ID NO: 19) | FGGGTKLEIK (SEQ ID NO: 20) |

**Example 2. Evaluation of effects on anti-coagulation of monoclonal antibody by APTT, PT.**

The inventors then tested the effects of the monoclonal antibody on activated partial thromboplastin time (APTT) and prothrombin time (PT) to evaluate its role in endogenous and exogenous coagulation pathways. The inventors added different concentrations of the monoclonal antibody (with a final concentration of 200, 100, 50, 25, 12.5, 6.25, 3.125, 1.56, 0.78 $\mu$g/ml) into the APTT or PT reaction system.

The experimental results showed that, compared with the control group, the FIXa-4 antibody significantly prolonged the APTT. Besides, as the concentration of FIXa-4 antibody increased, its prolonging effect on APTT was also enhanced, and the APTT could be prolonged to 88.8s at most, which was 3.5 times that of the control group at 25.5s, as shown in Figure 1B, IC50=7.705$\mu$g/ml. This prolongation effect is significantly higher than the 2.5 times APTT prolongation effect required by current antithrombotic drugs. When the antibody concentration was further increased, the APTT was not prolonged uncontrollably, but the ratio of APTT prolongation was limited within the range of 3.5 times. This result indicates that FIXa-4 has a large effective therapeutic concentration window, but does not increase the risk of bleeding. This can meet the needs in clinical application.

At the same time, the measurement of prothrombin time (PT) showed that the FIXa-4 antibody basically did not affect PT, as shown in Figure 1C.

Above experimental data indicate that FIXa-4 antibody specifically inhibits the endogenous coagulation pathway, but does not act on coagulation factors in exogenous coagulation pathways such as FX and FII and coagulation factors in common coagulation pathways.

**Example 3. FIXa-4 antibody does not directly bind to the enzyme catalytic active site of FIXa.**

In the endogenous coagulation pathway, FIXa acts as an enzyme that catalyzes FX to form FXa to mediate the coagulation cascade. Therefore, in order to study the anticoagulant mechanism of the FIXa-4 antibody, the present inventors first examined its effect on the catalytic activity of FIXa. The rate of an enzyme to catalyze substrate cleavage is used to reflect the catalytic activity of the enzyme. The inventors mixed different concentrations of FIXa-4 antibody (with a final concentration of 1000, 10, 1, 0.1, 0.01 $\mu$g/ml) and FIXa protein for 3 minutes, and then added it to the FIXa chromogenic substrate reaction system. The OD405nm was monitored in real time. $\Delta$OD405/min reflects the catalytic activity of the enzyme.

The experimental results showed that the FIXa-4 antibody has no significant effect on the enzymatic activity of FIXa, as shown in Figure 2.

This result indicates that the FIXa-4 antibody does not exert anticoagulant effects by directly binding to the catalytic active site of FIXa.

**Example 4. Prediction of the site between FIXa-4 antibody and FIXa**

In order to further study the anticoagulation mechanism of the FIXa-4 antibody, the inventors used protein docking method to predict the binding site of the FIXa-4 antibody and FIXa. Complete structure of full-length FIXa protein without experiments for determination is not available in the Protein Data Bank (PDB). Considering that the present disclosure mainly focuses on the effect of the antibody on the catalytic activity of FIXa, a 3D model of the catalytic domain of FIXa was established based on an inhibitor complex structure (PDBentry: 3LC3) of the catalytic domain of FIXa. Simultaneously, using antibody structure prediction tools, a 3D model of the binding region of the FIXa-4 antibody was established. The optimal ensemble of conformations for their binding was predicted through protein-protein docking. In this ensemble of conformations, a total of 979 docking conformations formed 30 clusters. By observing the representative conformations of each cluster, it was found that most binding conformations of FIXa and FIXa-4 antibody shared a binding surface, wherein the residues with higher contact frequency were listed in Table 2. In all conformations, the binding of the FIXa-4 antibody to FIXa does not obstruct the entry and exit of substrate to the catalytic site of FIXa (formed by the catalytic triad His57-Asp102-Ser195) (Figure 3A). At the same time, although conformational changes in FIXa and the antibody itself were not considered during the docking process, given that the binding site is distant from the catalytic site and the catalytic domain's three-dimensional structure is relatively stable, the possibility of large deformation influenced by binding is not high. Therefore, it can be preliminarily confirmed that the binding of FIXa-4 antibody does not directly affect the catalysis of FIXa.

On the other hand, these contact residues partially overlap with the previously predicted FIXa-FVIIIa binding interface [DeLano WL (2002) The PyMOL molecular graphics system] (Figure 3B, 3C). For instance, residues in FIXa listed in Table 1, such as Asn-c93, Lys-c132, Arg-c165, and Thr-c175, also bind to FVIIIa. Also, residues Ala-c95, Lys-c98, Asp-c164, Lys-c173, and Tyr-c177 are close in sequence to the predicted FIXa-FVIIIa binding residues, with less than 2 residues. In the 3D structure, these residues occupy a common surface located between the c170-helix and c131-helix of FIXa, precisely the critical region where FIXa binds to the 558-helix of FVIIIa. Therefore, the binding of FIXa-4 antibody may compete with FVIIIa for binding to FIXa. In summary, the prediction of the binding site of FIXa-4 antibody with the catalytic domain of FIXa suggests that the FIXa-4 antibody does not reduce its activity by directly binding to the catalytic active site of FIXa. Instead, it competitively binds to FIXa with FVIIIa, obstructing the formation of the FVIIIa-FIXa complex, inhibiting FVIIIa activation of FIXa in endogenous coagulation pathway, and consequently reducing the catalytic activity of FIXa.

Table 2. Most frequent residues of FIXa contacting with FIXa-4 antibody

| Residue | Frequency (%) |
|---|---|
| Asn93[a] | 62 |
| Ala95[b] | 34 |
| Lys98[b] | 32 |
| Lys126 | 87 |
| Asn129 | 45 |
| Lys132[a] | 48 |
| Asp164[b] | 30 |
| Arg165[a] | 90 |
| Lys173[b] | 33 |
| Thr175[a] | 33 |
| Tyr177[b] | 45 |
| Asn178 | 93 |
| Lys230 | 46 |
| Arg233 | 95 |
| Asn236 | 36 |

All the residues in the table are numbered according to chymotrypsin. The residues of FIXa within the proximity of 3Å to the antibody were considered as contact residues. The frequency was calculated as $\frac{\sum s^*}{\sum S}$, wherein the $\Sigma$ means summation, $S^*$ was the number of members of a docking cluster which contained the residue proximate to the antibody and s was the number of members of every cluster. Residues marked with "a" are the residues same to the predicted sites of FIXa binding to FVa. Residues marked with "b" are the residues in the vicinity of those binding residues by less than 2 residues in the sequence.

**Example 5 FIXa-4 antibody competes with FVIIIa for binding to FIXa**

As mentioned earlier, FIXa-4 competes with FVIIIa for binding to FIXa, so as the concentration of FVIIIa increases, the effect of FIXa-4 antibody will be weakened. Firstly, the inventors established an in vitro model that mimics the production of FXa by the FIXa-FVIIIa complex in vivo. In this system, PS/PC, FIXa, FVIIIa, and FX were added to react and produce FXa. Subsequently, FXa chromogenic substrates were added to determine the amount of FXa generated in the reaction.

The inventors found that the addition of different concentrations of FIXa-4 antibody (with a final concentration of 1560, 780, 390, 156, 78, 39, 15.6, 7.8 pM) to this system inhibited the generation of FXa in a dose-dependent manner (Figure 4A). When the concentration of FIXa-4 antibody reached 400pM, the production of FXa was almost completely inhibited. Furthermore, increasing the concentration of FVIIIa to different levels (with a final concentration of 1.1, 1.6, 2.5, 3.7, 5.6, 8.3, 12.5 nM) in this system showed that, with the elevation of FVIIIa concentration, the inhibitory effect of the antibody was gradually counteracted, reaching a correction rate of nearly 50% (Figure 4B).

This result proved that FIXa-4 exerts an anticoagulant effect by competing with FVIIIa for binding to FIXa.

In conclusion, the monoclonal antibody FIXa-4 targeting FIXa, obtained by the inventors, exhibits a significant anticoagulant effect. Its mechanism is to inhibit the formation of FVIIIa-FIXa complex by competing with FVIIIa to bind to the site on FIXa, thereby exerting anticoagulant effect.

The research results provide a new target for the treatment of thrombus, and also provide indirect evidence for the binding site of the interaction between FVIIIa and FIXa.

**Discussion**

The current anticoagulants have various limitations, including unpredictable pharmacokinetics, lack of reversibility, and, in some cases, immunogenicity. Developing safe and effective antithrombotic drugs remains a key focus in contemporary medical research. When selecting target coagulation factors, two important considerations should be considered. First, this pathway should be blocked before significant amplification of the coagulation cascade occurs. Second, the rate-limiting step should be targeted to provide effective anticoagulation within the broadest therapeutic range. FIXa is a critical coagulation factor in the endogenous coagulation pathway and is the only soluble form of coagulation protein. FIXa efficiently diffuses from tissue factor-bearing cells to platelets, serving as a crucial link between the initiation and amplification phases of the coagulation cascade.

Monoclonal antibodies have the characteristics of strong antigen-binding specificity and long half-life. With continuous development of monoclonal antibody technology, antibody drugs play a crucial role in disease prevention, diagnosis, and treatment. In the present disclosure, a novel anti-FIXa monoclonal antibody was obtained successively through hybridoma, antibody sequencing, cell expression and purification, and the antithrombotic properties of FIXa-4 were described through APTT and PT functional assays. Although the prolongation of APTT by FIXa-4 is dose-dependent, increasing the antibody concentration significantly does not result in uncontrolled prolongation but rather confines the APTT extension to approximately 3.5 times. This result indicates that FIXa-4 has a large effective therapeutic concentration window, but does not increase the risk of bleeding. This can meet the needs in clinical application.

When exploring its antithrombotic mechanism, the inventors found that the FIXa-4 antibody is different from the previously reported FIXa inhibitors in that it does not directly act on the catalytic active site of FIXa, but occupies most of the binding region between FIXa and FVIIIa, blocking the formation of the FIXa-FVIIIa complex, then affects the conversion of FXa to FXa and triggers anticoagulation. More interestingly, the FIXa-4 antibody substantially blocked the production of FXa in vitro, and supplementation with FVIIIa corrected this inhibition. That is, the antithrombotic effect of FIX-4 antibody was partially reversed with the increase of FVIIIa concentration. Compared with the existing antithrombotic drugs, the reversible antithrombotic effect needs to be considered in the development of novel safe and effective antithrombotic drugs. This feature can be used in clinical medication to prevent or treat uncontrollable bleeding side effects caused by excessive anticoagulation.

In summary, the present inventors have prepared a new type of antithrombotic antibody targeting the FIXa-FVIIIa binding site, providing new insights for treating thrombosis and shedding light on the still not entirely clear interaction between FVIIIa and FIXa binding sites.

Each reference provided herein is incorporated by reference to the same extent as if each reference was individually incorporated by reference. In addition, it should be understood that based on the above teaching content of the disclosure, those skilled in the art can practice various changes or modifications to the disclosure, and these equivalent forms also fall within the scope of the appended claims.

**Claims**

1. An antithrombotic monoclonal antibody or antigen-binding fragment thereof, wherein the antithrombotic monoclonal antibody or antigen-binding fragment thereof has a heavy chain CDR1 with the amino acid sequence of SEQ ID NO: 3, a heavy chain CDR2 with the amino acid sequence of SEQ ID NO: 4, a heavy chain CDR3 with the amino acid sequence of SEQ ID NO: 5; and a light chain CDR1 with the amino acid sequence of SEQ ID NO: 6, a light chain CDR2 with the amino acid sequence of SEQ ID NO: 7, a light chain CDR3 with the amino acid sequence of SEQ ID NO: 8.

2. The antithrombotic monoclonal antibody or antigen-binding fragment thereof according to claim 1, wherein, the monoclonal antibody comprises:

   (a) a heavy chain variable region with the amino acid sequence of SEQ ID NO: 1 and a light chain variable region with the amino acid sequence of SEQ ID NO: 2; or
   (b) a heavy chain variable region with more than 80% sequence identity to the amino acid sequence of SEQ ID NO: 1 and a light chain variable region with more than 80% sequence identity to the amino acid sequence of SEQ ID NO: 2, also having function of the monoclonal antibody of (a);

   preferably, the monoclonal antibody comprises: murine antibody, chimeric antibody or humanized antibody; or, the monoclonal antibody or the antigen-binding fragment thereof comprises: single-chain antibody, domain antibody, Fab fragment, Fab' fragment, Fd fragment, F(ab')2 fragment.

3. An isolated polynucleotide or a construct comprising the polynucleotide, the polynucleotide encodes the antithrombotic monoclonal antibody or antigen-binding fragment thereof according to any one of claims 1-2; preferably , the construct is an expression vector.

4. An antibody expression system, the expression system comprises the construct according to claim 3 or the exogenous polynucleotide according to claim 3 integrated in the genome; preferably, the expression system is a cell expression system.

5. A method for preparing the antithrombotic monoclonal antibody or antigen-binding fragment thereof according to any one of claims 1-2, comprising: expressing the antibody by the antibody expression system of claim 5 under conditions suitable for expression; preferably it also comprises purifying and isolating the antibody.

6. A fusion protein, comprising the antithrombotic monoclonal antibody or the antigen-binding fragment thereof according to any one of claims 1-2, and a fusion partner operably linked thereto; preferably, the fusion partner comprising: a protein or an active structural domain having an effect of extending half life in vivo, or a protein or an active structural domain having a synergistic function or binding effect on effectors; more preferably, the protein or active structural domain having an effect of extending half life in vivo comprises: an immunoglobulin Fc region, preferably a human immunoglobulin Fc region, serum albumin or a fragment thereof.

7. An immunoconjugate, wherein it comprises the antithrombotic monoclonal antibody or the antigen-binding fragment thereof according to any one of claims 1-2, or the fusion protein according to claim 6; and the functional molecules linked thereto; preferably, the functional molecules comprise: molecules targeting surface markers on blood cells, hydrophilic polymers or detectable markers.

8. A pharmaceutical composition, wherein the pharmaceutical composition comprises the antithrombotic monoclonal antibody or the antigen-binding fragment thereof according to any one of claims 1-2, the fusion protein according to claim 6, or the immunoconjugate according to claim 7; preferably, the pharmaceutical composition further comprises a pharmaceutically acceptable carrier.

9. A use of the antithrombotic monoclonal antibody or antigen-binding fragment thereof according to any one of claims 1-2, the fusion protein according to claim 6, or the immunoconjugate according to claim 7, or a pharmaceutical composition comprising them, in the manufacture of preparations, kits or drug kits for alleviating or treating thromboembolic diseases;
preferably, the thromboembolic diseases comprise: venous, arterial or capillary thrombosis, thrombosis in the heart, thrombosis during and/or after contacting blood with an artificial surface, interstitial lung disease, inflammation , neuro-inflammatory diseases, complement activation, fibrinolysis, angiogenesis, coagulation induced by FVIIIa-FIXa complex formation, coagulation induced by FX activation, coagulation induced by FIIa expansion, retinal vascular permeability-related diseases; preferably, diseases related to arterial or capillary thrombosis comprise: myocardial infarction, stroke, deep vein thrombosis, portal vein thrombosis, renal vein thrombosis, jugular vein thrombosis, cerebral venous sinus thrombosis, Budd-Chiari syndrome or Paget-Schroetter disease.

10. A kit or drug kit, comprising the antithrombotic monoclonal antibody or the antigen-binding fragment thereof according to any one of claims 1-2, the fusion protein according to claim 6, or the immunoconjugate according to claim 7 or pharmaceutical compositions comprising them.

A

Affinity assay of FIXa-4

B

APTT

C

PT

Figure    1

FIXa enzyme activity

Figure    2

A

B

C

Figure 3

FXa absolute production(nM)

antibody compete

FIXa-4 concentration log(pM)

A

Correction ratio of FIXa-4's inhibition (%)

FVIIIa correct

FVIIIa concentration(nM)

B

Figure 4

## INTERNATIONAL SEARCH REPORT

| International application No. |
|---|
| **PCT/CN2022/097412** |

| A. | CLASSIFICATION OF SUBJECT MATTER |
|---|---|
| | C07K 16/36(2006.01)i; C12N 15/13(2006.01)i; A61K 39/395(2006.01)i; A61P 7/02(2006.01)i |

According to International Patent Classification (IPC) or to both national classification and IPC

| B. | FIELDS SEARCHED |
|---|---|

Minimum documentation searched (classification system followed by classification symbols)

C07K; A61K; C12N; A61P

Documentation searched other than minimum documentation to the extent that such documents are included in the fields searched

Electronic data base consulted during the international search (name of data base and, where practicable, search terms used)

CNABS, DWPI, SIPOABS, CNKI, NCBI, ISI Web of Science, GenBank, 中国专利生物序列检索系统,Chinese Patent biological Sequence retrieval system: 抗体, 单抗, 凝血因子, antibody, monoclonal, factor, VIII, FVIII, IX, FIX, 本申请的SEQ ID NOs: 3-8

| C. | DOCUMENTS CONSIDERED TO BE RELEVANT | |
|---|---|---|
| Category* | Citation of document, with indication, where appropriate, of the relevant passages | Relevant to claim No. |
| A | CN 1501812 A (SMITHKLINE BEECHAM CORP.) 02 June 2004 (2004-06-02) entire document | 1-10 |
| A | CN 103396494 A (JIANGSU PROVINCIAL CENTER FOR DISEASE PREVENTION AND CONTROL) 20 November 2013 (2013-11-20) entire document | 1-10 |
| A | CN 101906160 A (SUZHOU ZELGEN BIOPHARMACEUTICALS CO., LTD.) 08 December 2010 (2010-12-08) entire document | 1-10 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **17 June 2022** | **18 August 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/097412** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

International application No.

**PCT/CN2022/097412**

| Patent document cited in search report | | | Publication date (day/month/year) | Patent family member(s) | | | Publication date (day/month/year) |
|---|---|---|---|---|---|---|---|
| CN | 1501812 | A | 02 June 2004 | ZA | 200209265 | B | 22 October 2003 |
| | | | | PL | 360060 | A1 | 06 September 2004 |
| | | | | BR | 0015872 | A | 03 August 2004 |
| | | | | MX | PA02011385 | A | 26 January 2004 |
| | | | | WO | 0187339 | A1 | 22 November 2001 |
| | | | | IL | 152831 | D0 | 24 June 2003 |
| | | | | NO | 20025463 | D0 | 14 November 2002 |
| | | | | HU | 0301804 | A2 | 28 August 2003 |
| | | | | ES | 2277597 | T3 | 16 July 2007 |
| | | | | US | 2005037006 | A1 | 17 February 2005 |
| | | | | CN | 101134107 | A | 05 March 2008 |
| | | | | KR | 20070116192 | A | 06 December 2007 |
| | | | | EP | 1282444 | A1 | 12 February 2003 |
| | | | | EP | 1825864 | A2 | 29 August 2007 |
| | | | | CA | 2411369 | A1 | 22 November 2001 |
| | | | | DE | 60032029 | D1 | 04 January 2007 |
| | | | | AU | 2000278584 | B2 | 16 November 2006 |
| | | | | IN | INPCT20021218DEL | A | 23 April 2010 |
| | | | | KR | 20030034071 | A | 01 May 2003 |
| | | | | AT | 345816 | T | 15 December 2006 |
| | | | | CZ | 20023778 | A3 | 13 August 2003 |
| | | | | NZ | 522632 | A | 27 May 2005 |
| | | | | JP | 2004516236 | A | 03 June 2004 |
| | | | | AU | 7858400 | A | 26 November 2001 |
| CN | 103396494 | A | 20 November 2013 | None | | | |
| CN | 101906160 | A | 08 December 2010 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LEONARD G PRESTA.** *Current Opinion in Immunology,* 2008, vol. 20, 460-470
- **ESOHE E.IDUSOGIE et al.** *J Immunol,* 2000, vol. 164, 4178-4184
- **RAPHAEL A. CLYNES et al.** *Nature Medicine,* 2000, vol. 6 (4), 443-446
- **PAUL R. HINTON et al.** *J Immunol,* 2006, vol. 176, 346-356
- **J. SAMBROOK.** Molecular Cloning: A Laboratory Manual. Science Press, 2002
- **DUNBAR J et al.** SAbPred: a structure-based antibody prediction server. *Nucleic Acids Res.,* 08 July 2016, vol. 44 (W1), W474-8